# EUROPÄISCHE PATENTSCHRIFT

(11) **EP 3 686 269 B1**
(45) Veröffentlichungstag und Bekanntmachung des Hinweises auf die Patenterteilung: **10.11.2021**
(21) Anmeldenummer: 19153761.2
(22) Anmeldetag: 25.01.2019
(51) Int. Cl.: C12M 1/24, C12M 3/04, C12M 1/26, C12M 1/00, C12M 1/12

(54) **ZELLKULTUR-FLASCHE**
CELL CULTURE BOTTLE
FLACON À CULTURE CELLULAIRE

(43) Veröffentlichungstag der Anmeldung: 29.07.2020
(73) Patentinhaber: Technische Hochschule Mittelhessen, 35390 Giessen (DE)
(72) Erfinder: Eichmann, Joel, 35390 Giessen (DE)
(74) Vertreter: Stumpf, Peter

(56) Entgegenhaltungen:
- EP-A1- 1 304 370
- EP-A2- 0 244 873
- WO-A2-2008/104586

## Beschreibung

Die vorliegende Erfindung betrifft eine Vorrichtung und ein Verfahren zur Kultivierung von Zellen. Die Vorrichtung stellt eine Rollerflasche mit einem verbesserten Oberflächen-Volumen-Verhältnis dar, in dem es bei gleicher Zeit möglich ist, mehr Zellen zu kultivieren. Zellkulturen finden breite Anwendung in der biologischen und medizinischen Forschung. Mittels Zellkultur können verschiedene biologische Stoffe, wie Proteine oder Antikörper in großer Menge hergestellt werden. Dies ist für Impfstoffherstellung, Diagnostik und immunologische Analyse wichtig.

### Beschreibung und Einleitung des allgemeinen Gebietes der Erfindung

Als Zellkultur wird die Kultivierung von Zellen in einem geeigneten Nährmedium außerhalb eines lebenden Organismus bezeichnet. Die Zellen können menschlichen oder tierischen Ursprung haben, aus Pflanzen, Algen oder Pilzen stammen oder Mikroalgen oder Bakterien sein. Die Zellen können auch Tumorzellen sein, oder Zellen, oder Zellen, die genetisch verändert sind (z.B. durch Einschleusung eines Plasmids als Vektor).

Zelllinien sind Zellen einer Gewebeart, die sich durch fortwährende Teilungen vermehren. Der Fachmann kennt nicht-immortalisierte Zelllinien, die nur eine begrenzte Teilungsrate aufweisen und er kennt immortalisierte also unsterbliche Zelllinien, die in der Zellkultur unbegrenzt am Leben gehalten werden können. Dies sind vor allem die Tumorzellen, die bekannteste nicht-immortalisierte Zellkultur wurde 1951 aus Cervixkarzinom entnommen und unter dem Namen HeLa bekannt.

Man unterscheidet zwischen Zellen, die in Suspensionskulturen vermehrt werden und Zellen, die verankerungsabhängig also adhärent wachsen. Suspensionszellen sind z.B. Lymphozyten die freischwimmend im Medium vorliegen.

Als Primärkultur bezeichnet man eine nicht-immortalisierte Zellkultur, die direkt aus einem Gewebe eines Organismus gewonnen wurde. Zur Gewinnung werden die Zellen z.B. mit Trypsin behandelt, das die Proteine abbaut, die den Zellverband aufrechterhalten. Somit werden Zellen vereinzelt. In einem speziellen Medium, das u.a. auch Wachstumsfaktoren enthält, beginnen sie sich durch Teilung zu vermehren. Die Kulturbedingungen für die einzelnen Zellen unterscheiden sich sehr stark. Dabei müssen spezielle Medien, pH-Wert, Nährstoff-Konzentration, CO₂-Gehalt und andere wichtige Parameter berücksichtigt werden, diese sind dem Fachmann bekannt und können in Standardwerken nachgelesen werden. Je nach Teilungsrate und Dichte der Zellen werden die Zellverbände gelöst und auf neue Gefäße verteilt, dieser Vorgang wird splitten oder Passage genannt. Da zu dicht wachsende Zellen eine Zellkontakthemmung aufweisen und nicht mehr weiterwachsen, müssen Zellen regelmäßig gesplittet werden. Dies betrifft insbesondere adhärent (auf Oberflächen) wachsende Zellen wie z.B. Fibroblasten oder Endothelzellen.

Typische kommerziell erhältliche Medien für Zellkulturen menschlichen Ursprungs sind RPMI-1640, Dulbecco's Modified Eagle Medium und Ham's F12. Zum Waschen der Zellen kennt der Fachmann Balaced Salt Solution, Hanks-Salze oder Earle Salze.

Einfache Gefäße für das Kultivieren von Zellen können Erlenmeyer-Kolben oder Petrischalen aus Glas oder Kunststoff sein. Die Gefäße werden mit einem bestimmten Volumen Flüssigmedium gefüllt, mit Zellen angeimpft und über einen bestimmten Zeitraum geschüttelt. Diese Gefäße weisen jedoch eine begrenzte Oberfläche auf, sodass das Zellwachstum stark limitiert ist. Weiterhin ist es schwer Kultivierungsparameter wie pH-Wert und Temperatur direkt im Gefäß zu überprüfen und zu regulieren. Dazu muss stets eine Probe entnommen werden, ein Vorgang, der immer eine Kontaminierungsgefahr darstellt, weil störende Keime wie Pilze oder Bakterien eindringen können. Auch der Wechsel von Medium ist mit einer Kontaminationsgefahr verbunden, weil das verbrauchte Medium mit einer Pipette abgenommen und das Gefäß mit frischem Medium aufgefüllt werden muss.

Für den industriellen Einsatz z.B. bei der Herstellung von Zellkulturen für Impfstoffe oder Stammzellen haben sich daher geschlossene Zellkulturschalen oder -flaschen mit verschiedenartigen Strukturen zur Oberflächenvergrößerung etabliert. Diese Oberflächenvergrößerungen werden meistens durch Rippen, auf denen die Zellen zusätzlich wachsen können, realisiert. Die Zellkulturschale oder -Flasche wird mit Flüssigmedium gefüllt, mit Zellen angeimpft und durch eine Rüttel-, Schüttel- oder Wippvorrichtung bewegt, damit die Zellen möglichst gut mit Nährmedium umspült werden und wachsen können. Allerdings ist es auch bei Zellkulturschalen oder - flaschen nicht möglich Kultivierungsparameter direkt im Gefäß zu überprüfen und zu regulieren. Die Zellkulturschale oder -Flasche muss für eine Probenentnahme geöffnet werden. Für die Kultivierung von Zelllinien wie Stammzellen haben sich die sogenannten Rollerflaschen etabliert. Dies sind runde Flaschen aus Glas oder Kunststoff mit Deckel, die eine innenliegende glatte oder gerippte Oberfläche aufweisen. Sie können kontinuierlich über eine Rollvorrichtung gedreht werden, so dass die im Inneren befindlichen Zellen kontinuierlich mit Medium umspült werden. Die innenliegenden Strukturen zur Oberflächenvergrößerung verhindern jedoch, dass alle Bereiche im Inneren, die von Zellen bewachsen werden, auch zuverlässig von Medium umspült werden. In diesen Bereichen, wo die Zellen nicht von Medium umspült werden, wachsen die Zellen langsamer oder sterben ab.

Besonders zuverlässig zur Kultivierung von Zellen sind Bioreaktoren oder sogenannte Fermentersysteme. Dies sind Behälter, die mit einem festen Deckel verschließbar sind in denen sich Medium und Zellen befinden. Fermenter weisen keine Oberflächenvergrößerungen auf, weil sich die Zellen nicht an den Wänden des Fermenters anheften, sondern auf Mikropartikeln wachsen. Die gleichmäßige Versorgung mit Medium ist in einem Fermenter sehr wichtig, häufig befindet sich im Deckel ein zentral angeordneter massiver Rührer, der mit dem Schließen des Fermenters in die Flüssigkeit eintaucht und Medium und die darin befindlichen Zellen bewegt bzw. rührt. Der Rührer ist meist ein Propellerrührer oder ein Paddelrührer. Bekannte Fermenter weisen verschiedene Zu- und Abläufe auf, über die Medium, Gase oder spezielle Nährstoffe zugeführt werden. Es ist üblich die Kultivierungsparameter wie pH-Wert und Temperatur über Sonden direkt im Gefäß zu überprüfen und zu regulieren. Nachteilig ist an diesen Fermentern, dass sie sehr teuer sind und deshalb nicht als Einwegobjekte benutzt werden. Aufgrund des Rührers, der auch am Boden des Behälters befestigt sein kann, ist nur begrenzter Raum für innenliegende Strukturen zur Oberflächenvergrößerung vorhanden.

Weitere Nachteile der Kultivierung im Fermenter sind die Scherkräfte, welche durch den Rührer entstehen. Diese entstehen in Abhängigkeit zur Rührgeschwindigkeit. Dadurch ergibt sich die Problematik, dass bei zu geringer Rührerdrehzahl keine homogene Durchmischung und ausreichender Gasaustausch gewährleistet ist, bei zu großer Rührerdrehzahl dagegen die Zellen wegen der Scherkräfte Schaden nehmen.

Sollen die Zellen von den Mikropartikeln gelöst werden, ergeben sich auch hier technische Schwierigkeiten. Zuerst müssen die Zellen von den Trägern abgelöst, und anschließend getrennt werden. Wird z.B. Trypsin für die Ablösung verwendet, sind längere Einwirkzeiten notwendig, wodurch die Zellen Schaden nehmen können. Anschließend müssen die Zellen von den Mikropartikeln separiert werden, was einen weiteren Prozessschritt mit allen damit verbunden Validierungs- und Investitionskosten darstellt.

Eine archimedische Schraube ist eine aus mehreren Windungen bestehende spiralig angeordnete Schraube zur Bewegung oder Förderung von festem oder flüssigem Material. Die Schraube dreht sich um eine massive Mittelachse bzw. einen Stab, wobei mehrere Kammern gebildet werden. Durch die Rotation der Schraube bewegen sich alle Kammern in Richtung des Schraubenendes und transportieren das feste oder flüssige Material. Die Kammern sind nach oben und unten durch jeweils einen Blattabschnitt der Spirale begrenzt. Am oberen Ende der Schraube läuft das Material aus der sich auflösenden Kammer aus, am Schraubenanfang entsteht eine neue Kammer, die sich mit Material aus einem Zulauf füllt.

Physikalisch ist eine archimedische Schraube definiert durch folgende Größen: Rohrdurchmesser der massiven Mittelachse, der Schraubendurchmesser, Aufstellwinkel der gesamten Schraube relativ zur Standoberfläche, die konstruktive Förderhöhe, Flüssigkeitsspiegeldifferenz, die maximale Förderhöhe, erforderliche hydraulische Förderhöhe, Gangzahl, beschaufelte Länge und Steigung.

Die archimedische Schraube ist als Rührervorrichtung in Bioreaktoren bzw. Fermentern aus dem Stand der Technik bekannt und wird entweder als fest montiertes Bauteil im Boden des Bioreaktors befestigt oder sie ist am Deckel befestigt. Sie sorgt für eine homogene Vermischung des Flüssigkeitsvolumens, also Medium und darin befindlichen Zellen. Die Bioreaktoren sind meist aus Metall (Edelstahl) oder Glas gefertigt, die archimedische Schraube ist ebenfalls aus Metall gefertigt, damit eine einfache Reinigung oder Sterilisierung möglich ist. Bioreaktoren, die so gefertigt sind, sind aufwendig und teuer in der Herstellung. Sie sind keine Einwegmaterialien. Da in der Biotechnologie verstärkt gentechnisch veränderte Zellen verwendet werden, besteht ein erhöhter Bedarf an Einmalmaterialien. Die aufwendigen Reinigungsprozeduren entfallen so und Rückstände können leichter und sicherer entsorgt werden. EP1304370 offenbart eine Zellkultur-Flasche für adhärente Zellen (Abbildung 3). Die Zellkultur-Flasche weist ein Mittelrohr und drehbare Platten, die um das Mittelrohr angeordnet sind, auf.

### Aufgabe

Aufgabe der vorliegenden Erfindung ist es eine verbesserte Zellkultur-Flasche für adhärente Zellen bereitzustellen, die eine größere Oberfläche zum Anhaften der Zellen aufweist und die es ermöglicht, dass die Zellen während der Wachstumszeit zuverlässig mit Medium umspült werden.

### Lösung der Aufgabe

Diese Aufgabe wird erfindungsgemäß gelöst durch eine Vorrichtung gemäß Anspruch 1. Vorteilhafte Ausgestaltungen der Vorrichtung sind den Unteransprüchen zu entnehmen.

Die Erfindung betrifft eine neuartige Zellkultur-Flasche 1 für adhärente Zellen (z.B. humane mesenchymale Stammzellen).

Die erfindungsgemäße Zellkultur-Flasche 1 umfasst folgende Bauelemente: Das Gefäß 10 bildet die äußere Hülle der Zellkultur-Flasche 1. Es weist bevorzugt eine Wandstärke von 0,1 bis 1 mm und einen Durchmesser D von vorzugsweise 5 cm bis 30 cm.

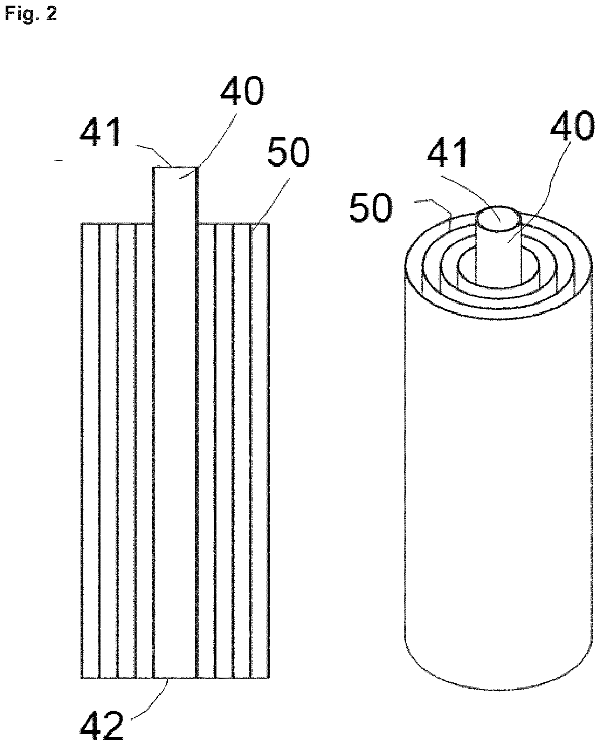

Das Gefäß 10 ist einstückig ausgebildet und weist drei Teilbereiche auf: einen Bodenbereich 12, einen Deckelbereich 14 und Zylinderbereich 16, der zwischen Bodenbereich 12 und Deckelbereich 14 angeordnet ist. Dabei ist der Zylinderbereich 16 zylinderförmig ausgebildet. Das Gefäß 10 weist im Deckelbereich 14 eine Öffnung auf. Die dem Deckelbereich zugewandte Seite des Zylinderbereiches 16 ist definitionsgemäß die Oberseite des Zylinderbereichs. Die dem Deckelbereich abgewandte Seite des Zylinderbereiches 16 ist definitionsgemäß die Unterseite des Zylinderbereichs. Der Bodenbereich 12 ist offen oder geschlossen ausgebildet. Die erfindungsgemäße Zellkultur-Flasche 1 umfasst eine im Zylinderbereich 16 des Gefäßes 10 angeordnete archimedische Schraube 30 mit einem entlang ihrer Mittelachse angeordneten flüssigkeitsdurchströmbaren Mittelrohr 40 sowie wenigstens einer konzentrisch um das Mittelrohr 40 angeordneten Wand 50. Das Mittelrohr 40 dient dazu, dass mindestens eine Sonde für die Überwachung der Wachstumsbedingungen der Zellen eingebracht werden kann oder Fluid eingeleitet bzw. entnommen werden kann. Die archimedische Schraube 30 bildet mehrere schraubenartige Windungen um das Mittelrohr 40. Die archimedische Schraube 30 ist einstückig mit dem Zylinderbereich 16 des Gefäßes 10 und der wenigstens einen Wand 50 verbunden. Alternativ können auch mehrere Wände 50 verwendet werden (siehe Abbildung Fig.2). Die archimedische Schraube 30 und die wenigstens eine Wand 50 sind einstückig miteinander verbunden. Weiterhin sind die archimedische Schraube 30 und die wenigstens eine Wand 50 so angeordnet, dass ein Fluid (z.B. Medium oder Gas) entlang der archimedischen Schraube 30 von der Unterseite des Zylinderbereiches 16 in den Deckelbereich 14 des Gefäßes 10 gelangen kann, wenn sich die erfindungsgemäße Zellkultur-Flasche 1 dreht oder sie gedreht wird.

In die erfindungsgemäße Zellkultur-Flasche 1 wird insbesondere in den Zylinderbereich 16 Fluid (z.B. Medium oder Gas) mit den zu kultivierenden Zellen eingebracht, so dass sich bei geeigneten Bedingungen das Fluid mit den Zellen im Inneren des Gefäßes 10, also an der wenigstens einen Wand 50, an der archimedischen Schraube 30, am Mittelrohr 40 und an der Innenseite des Gefäßes 10 verteilt. Dies sind die inneren Oberflächen der Zellkultur-Flasche 1. Die Zellen haften an diesen inneren Oberflächen an und wachsen. Gegenüber bekannten Vorrichtungen vergrößern die wenigstens eine Wand 50 und die archimedische Schraube 30 die innere Oberfläche der Zellkultur-Flasche 1, sodass mehr Zellen pro Flasche im Inneren des Gefäßes 10 anheften und wachsen können.

Die archimedische Schraube 30 füllt den gesamten Innendurchmesser des Gefäßes 10 aus. Das Mittelrohr 40 ist parallel zur Wand des Zylinderbereiches 16 angeordnet und weist vorzugsweise einen Außendurchmesser d von 0,5 bis 2,0 cm auf. Der Innendurchmesser beträgt bevorzugt 0,48 cm bis 1,98 cm. Das Mittelrohr 40 kann aus dem Deckelbereich 12 des Gefäßes 10 herausragen. Eine erste Öffnung 41 befindet sich am oberen Ende des Mittelrohrs 40 an der Oberkante des Deckelbereiches 14. Alternativ befindet sich die erste Öffnung 41 in dem Bereich des Mittelrohrs 40, der aus dem Deckelbereich 12 herausragt

In einer alternativen Ausführungsform der erfindungsgemäßen Zellkultur-Flasche 1 umfasst diese im Deckelbereich 14 noch einen separaten Deckel zum Verschluss und zur Vermeidung einer Kontamination beim Transport. Dieser Deckel ist so ausgebildet, dass er die Öffnung 41 verschließen kann. Der Deckel weist dazu einen leicht größeren Durchmesser auf, als der Deckelbereich 14 des Gefäßes 10, sodass die Verbindung zwischen dem Gefäß 10 und dem Deckel über eine Steckverbindung erfolgt. Alternativ weisen Deckel und Gefäß 10 Gewinde auf, so dass die Verbindung zwischen dem Gefäß 10 und dem Deckel über verschrauben erfolgt. Der Deckel weist vorzugsweise einen Filter auf, welcher einen Gasaustausch ermöglicht, jedoch vor Kontaminationen schützt, z.B. einen Sterilfilter.

Weiterhin ist es möglich, die erfindungsgemäße Zellkultur-Flasche 1 über das Mittelrohr 40 und die erste Öffnung 41 an ein Kultivierungssystem anzuschließen, z.B. zur Zu- bzw. Ableitung von Fluid oder Zellen. Dies erfolgt z.B. über ein Schlauchsystem, das in Verbindung mit der erfindungsgemäßen Zellkultur-Flasche 1 steht. Weiterhin weist das Mittelrohr 40 eine zweite Öffnung 42 im Inneren des Gefäßes 10 an der Unterseite des Zylinderbereiches auf. Durch die erste Öffnung 41 oder die zweite Öffnung 42 können Fluid (z.B. Medium oder Gas) mit den zu kultivierenden Zellen in das Innere des Gefäßes 10 gelangen und auch wieder entnommen werden.

Die Steigung der archimedischen Schraube beträgt bevorzugt 2 mm bis 10 mm.

Mit Steigung ist hier der Abstand zwischen zwei Ebenen der archimedischen Schraube 30 gemeint. Der Steigungswinkel α der archimedischen Schraube 30 beträgt bevorzugt ca. 2° bis 5°.

Die Zellkultur-Flasche 1 wird mit einem gewissen Inokulum an Fluid (z.B. Medium oder Gas) mit den zu kultivierenden Zellen befüllt. Vorzugsweise erfolgt dies über das Mittelrohr 40, sodass Fluid und Zellen in das Innere des Gefäßes 10 gelangen und dessen innere Oberflächen benetzen können. Die Zellen können sich dabei an den konzentrischen Wänden 50 und an der innenliegenden archimedischen Schraube 30 festsetzen und diese als Wachstumsoberfläche nutzen. Durch Drehung der Zellkultur-Flasche 1 werden alle Bereiche des Inneren des Gefäßes 10 benetzt und es kommt zu einer optimalen Durchmischung des Fluid (z.B. Medium oder Gas) mit den zu kultivierenden Zellen.

Das Gefäß 10 weist bevorzugt außerhalb des Mittelrohres 40 eine Öffnung 11 im Deckelbereich 14 auf. Über diese Öffnung 11 des Gefäßes 10 können Zellen und verbrauchtes Fluid (z.B. Medium oder Gas) entnommen werden.

Der bevorzugte Ausstellwinkel β des Gefäßes 10 und damit der archimedischen Schraube 30 beträgt zwischen 15° und 60° bevorzugt weniger als 45°. Ein geringerer Ausstellwinkel ist vorteilhaft, um mit weniger (oft sehr teurem) Fluid (z.B. Medium) die Mindestfüllhöhe zu erreichen. Der Winkel kann auch kleiner als 30° sein, entscheidend ist, dass bei Drehung der Zellkultur-Flasche 1 Fluid ins Innere des Gefäßes 10 gefördert wird und dabei Gas verdrängt, bevor es durch das Mittelrohr 40 zurückfließt.

Die Schichtdicken der Bereiche der erfindungsgemäßen Zellkultur-Flasche 1 betragen vorzugsweise zwischen 0,1 mm und 2,0 mm. Die minimale bzw. maximale mögliche Schichtdicke ist dabei auch vom Herstellungsverfahren abhängig. Wenn die erfindungsgemäße Zellkultur-Flasche 1 mehrere Wände 50 umfasst, beträgt deren Abstand vorzugsweise zwischen 3 mm und 10 mm. Als vorteilhaft hat sich eine Anzahl von 2 bis 16 Wänden erwiesen.

In einer zweiten Ausführungsform der erfindungsgemäßen Zellkultur-Flasche 1 weist diese noch eine Beschichtung auf. Diese ist auf der Oberfläche der archimedischen Schraube 30, der wenigstens einen Wand 50 und/oder der Innenseite des Gefäßes 10 angebracht. Sie dient dazu, dass sich die Zellen besser an den inneren Oberflächen der Zellkultur-Flasche 1 anhaften und diese möglichst vollständig besiedeln können. Die Beschichtung umfasst Polysaccharide und/oder Peptide. Alternativ kann die Oberfläche mittels eines Plasmas hydrophilisiert werden.

In einer dritten Ausführungsform der erfindungsgemäßen Zellkultur-Flasche 1 weist diese einen geschlossenen Bodenbereich 12 auf, sodass die Zellkultur-Flasche 1 im unteren Bereich geschlossen ausgebildet ist. Der Bodenbereich 12 ist einstückig mit dem Zylinderbereich 16 und dem Deckelbereich 14 ausgebildet.

In einer vierten Ausführungsform der erfindungsgemäßen Zellkultur-Flasche 1 weist diese einen Drehmechanismus zum Drehen des Gefäßes 10 auf. Der Drehmechanismus ist z.B. ein Motor. Er ist am Bodenbereich 12 anbringbar. Die Verbindung des Drehmechanismus mit dem Bodenbereich 12 erfolgt z.B. mit mindestens einem Dorn, mittels mindestens einer Ein- oder Ausstülpung oder mittels Magneten oder ähnlichen Befestigungsmöglichkeiten. Der Drehmechanismus ist in der Lage, das Gefäß 10 mit der archimedischen Schraube 30 in einer beliebigen Geschwindigkeit zu drehen, so dass bei Verwendung mit adhärenten Zellen und Fluid im Inneren des Gefäßes 10 möglichst große Anteile der inneren Oberfläche mit Fluid umspült werden, um so ein optimales Wachstum der adhärenten Zellen zu gewährleisten.

In einer fünften Ausführungsform der erfindungsgemäßen Zellkultur-Flasche 1 weist diese mindestens einen Filter im Mittelrohr 40 auf. Der Filter ist für Gase durchlässig, schützt aber das Innere der Zellkultur-Flasche 1 vor Kontaminationen. Er ist z.B. ein Sterilfilter. Dieser kann dabei direkt im Mittelrohr oder jeder der Öffnungen 11, 41, 42 angeordnet sein.

In einer sechsten Ausführungsform weist das Mittelrohr 40 eine dritte Öffnung 43 auf. Diese befindet sich an der Kontaktstelle des Mittelrohres 40 mit dem Deckelbereich 14 des Gefäßes 10. Diese Öffnung 43 dient als Überlauf, sodass Fluid (z.B. Medium oder Gas) mit den zu kultivierenden Zellen, wenn es den Deckelbereich 14 des Gefäßes 10 erreicht, über das Mittelrohr 40 wieder in den Bodenbereich 12 des Gefäßes 10 zurückfließen kann. In dieser Ausführungsform kann die Öffnung 11 entfallen.

Weiterhin ist es möglich die erfindungsgemäße Zellkultur-Flasche 1 in einem Kultivierungssystem zu verwenden. Dabei erfolgen der Zustrom und/oder die Entnahme von Fluid (z.B. Medium oder Gas) mit den zu kultivierenden Zellen über das Mittelrohr 40.

Die erfindungsgemäße Zellkultur-Flasche 1 erfüllt innerhalb eines Kultivierungssystems die Aufgabe eines Einwegreaktors bzw. eines Einweg-Rührreaktors. Dies ist vorteilhaft für Produktionsprozesse von scherempfindlichen Zellen (Suspensions- und adhärente Kulturen) und pharmazeutischen Produkten, ebenso wie zur Herstellung von Medien, Inokulum und Mischprozessen. Die erfindungsmäße Zellkultur-Flasche 1 ist dabei als Einwegflasche ausgebildet.

Der Vorteil der Verwendung von Einwegsystemen liegt in der Elimination von Sterilisation in Place (SIP), Cleaning in Place (CIP) und somit der Reduktion von Prozesszeiten. Auch wird das Risiko von Kreuzkontaminationen reduziert, der Qualifizierungs- und Validierungsaufwand eines Prozesses wird reduziert, was in der Gesamtbetrachtung Kostenersparnisse von bis zu 50% ermöglich. Es ist keine CIP-Validierung für ein externes Abtrennsystem notwendig.

Bei Bewegung des Gefäßes 10 mit der archimedischen Schraube 30 findet eine Bewegung und gleichmäßiger Austausch von Fluid (z.B. Medium oder Gas) mit den zu kultivierenden Zellen statt, so dass die Zellen über den Kultivierungszeitraum gleichmäßig gut versorgt werden. Diese Funktion entspricht einem Rührer in einem Rührreaktor. Durch das innenliegende senkrecht stehende Mittelrohr 40 kann von außen einfach und auf sterile Weise eine oder mehrere Messsonden zum Messen von Temperatur, pH-Wert, Gasgehalt o.ä. eingeführt werden, frisches Fluid oder zusätzliche Stoffe wie Trypsin hinzugefügt werden und/oder verbrauchtes Fluid abgenommen werden, um den Kultivierungsprozess (also Zellen und/oder Fluid) zu überwachen, zu messen, zu steuern und zu regeln. Ausleitung und Einleitung des Fluids sind zeitlich separiert durchführbar. Die Einleitung von frischem Fluid kann über eine Sonde im Mittelrohr 40 erfolgen, sodass frisches Fluid das Gefäß 10 über die zweite Öffnung 42 des Mittelrohres 40 erreicht. Die Entnahme von verbrauchtem Fluid und/oder Zellen kann über eine zweite Sonde über die Öffnung 11 im Deckelbereich 14 oder die Öffnung 41 erfolgen. Diese Anordnung ermöglicht eine Automatisierung des Kultivierungsprozesses. Die Zellvermehrung wird deutlich gesteigert, bei gleichzeitiger Material- und Personalersparnis. Besonders vorteilhaft ist es, dass durch die erfindungsgemäße Anordnung der Bauteile der Zellkultur-Flasche 1, eine wesentlich geringere Impfdichte an zu kultivierenden Zellen erforderlich ist, weil die Zellen zuverlässig mit Fluid umspült werden und eine größere Oberfläche für das Wachstum vorhanden ist.

Das Kultivierungssystem umfasst dabei bevorzugt noch weitere Bauelemente:
Ein Messmittel zur Erfassung verschiedener Parameter, die für eine erfolgreiche Zellkultivierung wichtig sind, z.B. pH-Wert, Sauerstoffpartialdruck, CO₂-Partialdruck, Temperatur als Messdaten. Messmittel können sein z.B. eine pH-Messsonde, eine Sauerstoffsonde, eine Temperaturmesssonde und/oder weitere Messsonden. Die Messung dieser Parameter kann dabei über Impedanzspektroskopie erfolgen. Dies ermöglicht es, die Zellzahl der Zelllinie, deren Vitalität und eventuell Produktionsaktivität zu kontrollieren und so den gesamten Kultivierungsverlauf zu automatisieren, sodass ein exaktes Ende des Kultivierungsvorganges festlegbar ist.

Das Kultivierungssystem umfasst weiterhin ein Auswertemittel, um die Messdaten des Messmittels auszuwerten und mit Referenzdaten zu vergleichen und ein Abweichen des Messdaten festzustellen. Das Auswertemittel wertet zur Erkennung von Problemen bei der Zellkultivierung besonders vorteilhafterweise die Messparameter gemeinsam aus, sodass die Zuverlässigkeit gesteigert und die Wahrscheinlichkeit einer Fehlfunktion durch eine mangelhafte Messdatenbasis verringert wird. Die Übertragung der vom Messmittel erzeugten Messdaten an ein Auswertemittel kann kabelgebunden oder drahtlos erfolgen.

Vorzugsweise umfasst das Auswertemittel noch eine Datenbank oder einen Zugriff auf eine Datenbank oder auf hinterlegte Referenzdaten der Messdaten. Mit diesen Referenzdaten können dann die erfassten Messdaten im Inneren der Zellkultur-Flasche 1 verglichen werden. Die Abweichungen werden erfasst, gespeichert oder versendet z.B. einer Steuerungseinheit, die den Gesamtprozess steuert und Fehlfunktionen entgegenwirkt. Weiterhin umfasst das Kultivierungssystem vorzugsweise ein Ausgabemittel zur Ausgabe der vom Messmittel gemessenen Messdaten und/oder der bestimmten Abweichungen zu den Referenzdaten.

Die erfindungsgemäße Zellkultur-Flasche 1 ist bevorzugt aus einem Material gefertigt, welches zur additiven Fertigung geeignet ist, wie z.B. Kunststoff z.B. Acrylnitril-Butadien-Styrol (ABS), Acrylester-Styrol-Acrylnitril (ASA), High-Impact Polystyrene (HIPS), thermoplastisches Elastomere (TPE), Polycarbonate (PC), Polylactide (PLA), Polyethylenterephthalat (PET), Polyethylenterephthalat Glycol (PETG), Polymethylmethacrylat (PMMA) oder Polyvinylalkohol (PVA oder PVOH). Alternativ wird als Material Harz verwendet. Harze sind nach ISO 4618:2014 durch Polymerisations-, Polyadditions- oder Polykondensationsreaktionen synthetisch hergestellte Stoffe. Gemäß den Konventionen der IUPAC sind dies weiche Feststoffe oder hochviskose Substanzen, die üblicherweise Prepolymere mit reaktiven funktionellen Gruppen enthalten. Synthetisch hergestellte Harze bestehen bei der Verarbeitung in der Regel aus zwei Hauptkomponenten. Die Vermischung beider Teile (Harz und Härter) ergibt eine reaktionsfähige Harzmasse. Bei der Härtung steigt die Viskosität an und nach abgeschlossener Härtung erhält man einen unschmelzbaren Kunststoff.

Die Herstellung der erfindungsgemäßen Zellkultur-Flasche 1 erfolgt mittels additiver Fertigungsverfahren wie z.B. Selective Laser Sinthering (SLS) / Selektives Laserschmelzen, Fused Deposition Modeling (FDM), Stereolithografie (SLA / DLP). Diese Verfahren sind dem Fachmann bekannt. Mit ihnen ist es möglich, Überhänge und Hohlräume zu drucken, ohne dass ein Stützmaterial verwendet und dieses nach dem Druck entfernt werden muss. Die genannten Verfahren sind dem Fachmann bekannt. Es ist bevorzugt die erfindungsgemäße Zellkultur-Flasche 1 sterilisiert herzustellen. Für FDM gibt es bereits kommerziell erhältliches steriles Material und das Verfahren wird steril durchgeführt, so dass die erfindungsgemäße Zellkultur-Flasche 1 steril hergestellt wird. Auch bei SLA und SLS führt die Herstellung zu einer sterilen Flasche. Alternativ wird die erfindungsgemäße Zellkultur-Flasche 1 erst nach der Fertigung sterilisiert, z.B. über Gamma-Sterilisation.

Ein Verfahren zur Kultivierung von Zellen in einer erfindungsgemäßen Zellkultur-Flasche 1 umfasst wenigstens folgende Schritte:
a) Befüllen der Zellkultur-Flasche 1 mit Fluid (Medium, Flüssigkeit, Gas)
b) Zugabe der zu kultivierenden Zellen in die Zellkultur-Flasche 1
c) Drehen der Zellkultur-Flasche 1 so dass sich das Fluid verteilt und die Zellen bedeckt, so dass sich die Zellen im Gefäß 10, in der archimedischen Schraube 30, an der wenigstens einen Wand 50 und dem Mittelrohr 40 festsetzen und teilen können
d) Entfernen des Fluids bei einer bestimmten Zelldichte
e) Zugabe eines Puffers in die Zellkultur-Flasche 1, um die Zellen zu waschen.
f) Ablösen der Zellen vom Gefäß 10, der archimedischen Schraube 30, der wenigstens einen Wand 50 und dem Mittelrohr 40
g) Stoppen der Ablösereaktion durch Zugabe von frischem Fluid
h) Entnahme des Fluids mit losgelösten kultivierten Zellen

Das Befüllen der Zellkultur-Flasche 1 in Schritt a) erfolgt z.B. mittels einer serologischen Pipette, wobei über das Mittelrohr 40 so viel Fluid in die Zellkultur-Flasche 1 eingefüllt wird, dass bei einer Rotation der Zellkultur-Flasche 1 das Fluid soweit in Richtung Deckelbereich 14 gefördert wird, dass das geförderte Fluid innerhalb der archimedischen Schraube das dort verbleibende Gas verdrängt.

Das Entfernen des Fluids in Schritt d) erfolgt z.B. mittels einer serologischen Pipette. Der Puffer in Schritt e) ist z.B. PBS. Er ist gut zum Waschen der Zellen geeignet. Das Ablösen der Zellen in Schritt f) erfolgt durch Zugabe von Trypsin oder einem vergleichbaren Enzym zum Ablösen der Zellen z.B. mittels einer serologischen Pipette.

### Ausführungsbeispiele

**Ausführungsbeispiel 1:** Verwendung der erfindungsgemäßen Zellkultur-Flasche 1 im Stand-alone Betrieb:
Die Zellkultur-Flasche 1 wird in aseptischer Umgebung (z.B. Sicherheitswerkbank) mit geeignetem Fluid für die zu kultivierenden Zellen und einer Starterkultur aus zu kultivierenden Zellen befüllt. Anschließend wird die Zellkultur-Flasche 1 mittels Drehmechanismus gedreht oder auf eine Drehapparatur gelegt. Eine solche Drehapparatur umfasst bevorzugt mindestens zwei Rollen, auf welche die Zellkultur-Flasche 1 gelegt wird. Die beiden Rollen drehen sich, wodurch die darauf liegende Zellkultur-Flasche 1 in entgegengesetzte Richtung in Rotation versetzt wird. Die Drehapparatur liegt waagerecht oder bevorzugt in einem Anstiegswinkel β des Gefäßes 10. Eine für die Zellen geeignete Rotationsgeschwindigkeit ist an der Drehapparatur einstellbar. Die Drehapparatur kann wie ein Brutschrank eingehaust sein, sodass wichtige Parameter wie z.B. Temperatur, O₂-Partialdruck und CO₂-Partialdruck im Inneren konstant gehalten werden können. Die Zellen, die kultiviert werden, sind adhärente Zellen, wie z.B. humane mesenchymale Stammzellen. Die Zeitdauer der Kultivierung ist je nach Zellart unterschiedlich, im allgemeinem dauert sie bis zu einer bestimmten Zelldichte, die dem Fachmann bekannt ist.

Die Entnahme der kultivierten Zellen ist dem Fachmann bekannt und erfolgt nach Standardvorgaben.

**Ausführungsbeispiel 2:** Verwendung der erfindungsgemäßen Zellkultur-Flasche 1 in einem klassischen Bioreaktor, z.B. in einem schräg stehenden Bioreaktor, an Stelle eines Rührers.

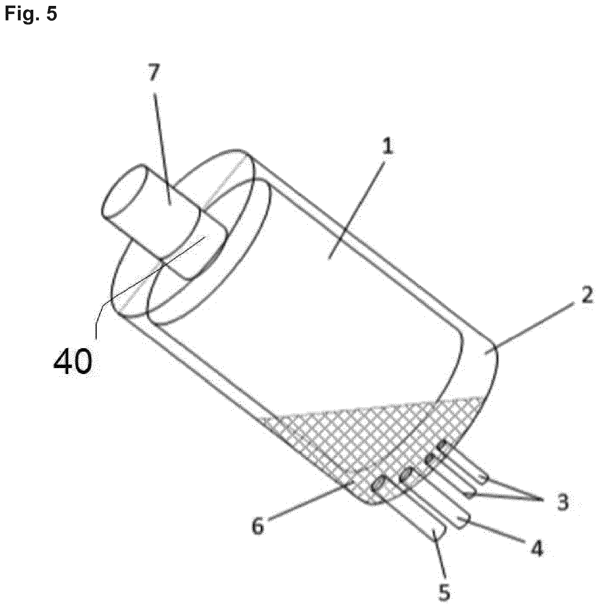

In dieser Ausführungsform ist die Zellkultur-Flasche 1 im Bodenbereich 12 offen, sodass die Schaufel der archimedischen Schraube 30 offen liegt. Die Zellkultur-Flasche 1 befindet sich als Gesamtes vollständig im Inneren eines Reaktorkessels 2 eines Bioreaktors (siehe Figur 5). Übliche Bioreaktoren und Reaktorkessel sind dem Fachmann bekannt. Der Reaktorkessel 2 wird wie üblich mit Fluid und Zellen befüllt, beheizt und begast, damit die geeigneten Reaktionsbedingungen für das Wachstum der zu kultivierenden Zellen erreicht werden. Das Fluid und die zu kultivierenden Zellen werden von außen in den Reaktorkessel 2 zu- und abgeführt, so dass durch Rotation der Zellkultur-Flasche 1 auch Fluid und Zellen ins Innere des Gefäßes 10 gelangen. Die erfindungsgemäße Zellkultur-Flasche 1 übernimmt im Inneren des Reaktorkessels 2 die Funktion eines Rührers. Der Reaktorkessel 2 wird soweit mit Fluid befüllt, so dass dieses durch Rotation der Flasche ins Innere des Gefäßes 10 gelangt. Die Rotation erfolgt indem ein externen Drehmechanismus an der Zellkultur-Flasche 1 angebracht wird, z.B. über das Mittelrohr 40. Dieser kann z.B. wie bei Rührkesselreaktoren für die Rührerwelle üblich, über eine Steckverbindung außerhalb des Kessels angebracht sein (Abbildung Figur 5). Der Bioreaktor ist mit den dem Fachmann bekannten Sonden 3 für pH-Wert, Temperatur, O₂-Partialdruck, CO₂-Partialdruck etc. ausgestattet und mit einem Steuerungssystem z.B. einem Prozessleitsystem verbunden. Darüber können die Kultivierungsbedingungen im Reaktor jederzeit gemessen und nachjustiert werden. Die Kultivierung endet, wenn die gewünschte Zelldichte in der erfindungsgemäßen Zellkultur-Flasche 1 erreicht ist. Dann wird das Fluid aus dem Reaktorkessel 2 und auch aus dem Inneren der erfindungsgemäßen Zellkultur-Flasche 1 entfernt und ein geeigneter Puffer wie z.B. PBS zugeführt, um die Zellen zu waschen. Anschließend wird unter Rotation der erfindungsgemäßen Zellkultur-Flasche 1 ein Enzym, wie beispielsweise Trypsin in den Reaktorkessel 2 und die erfindungsgemäße Zellkultur-Flasche 1 eingefüllt, um die Zellen von der Oberfläche im Inneren der erfindungsgemäßen Zellkultur-Flasche 1 zu lösen. Danach wird in den Reaktorkessel 2 und die erfindungsgemäße Zellkultur-Flasche 1 frisches Fluid zugeführt, so dass die Zellen in einer Suspension vorliegen und für die später vorhergesehene Anwendung geerntet werden können.

### Abbildungslegenden und Bezugszeichenliste

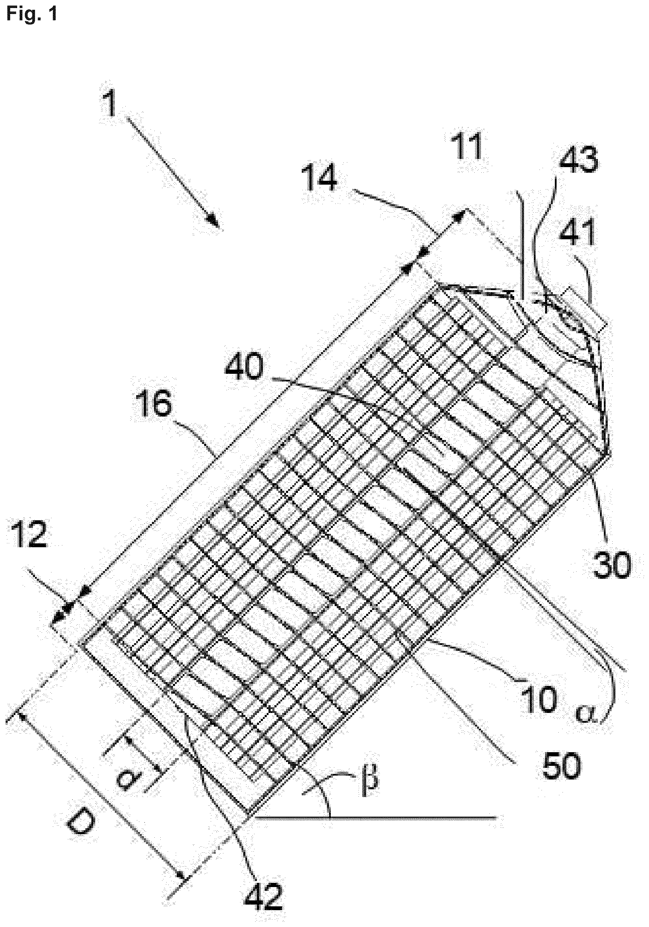

Fig. 1 zeigt beispielhaft eine Ausführungsform der erfindungsgemäßen Zellkultur-Flasche 1. Der gezeigte geschlossene Bodenbereich 12 ist dabei optional. Weiterhin ist die dritte Öffnung 43 des Mittelrohrs 40 optional.
Fig. 2 zeigt eine beispielhafte Anordnung mehrerer Wände 50 um das Mittelrohr 40. Die übrigen Bauteile der Zellkultur-Flasche 1 sind aus Übersichtlichkeitsgründen in dieser Abbildung nicht gezeigt.

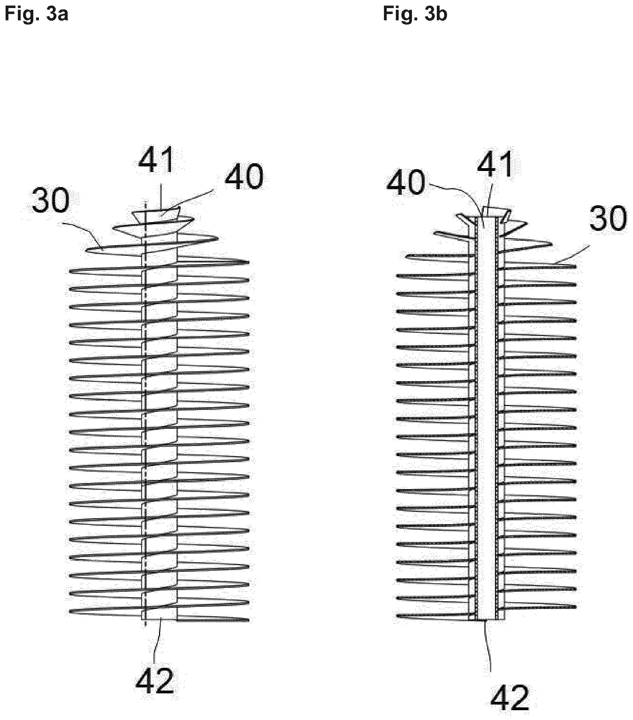

Fig. 3 zeigt beispielhaft in einer Seitenansicht Fig. 3a eine archimedische Schraube 30 mit einem Mittelrohr 40. Die Fig. 3b zeigt den Längsschnitt an.

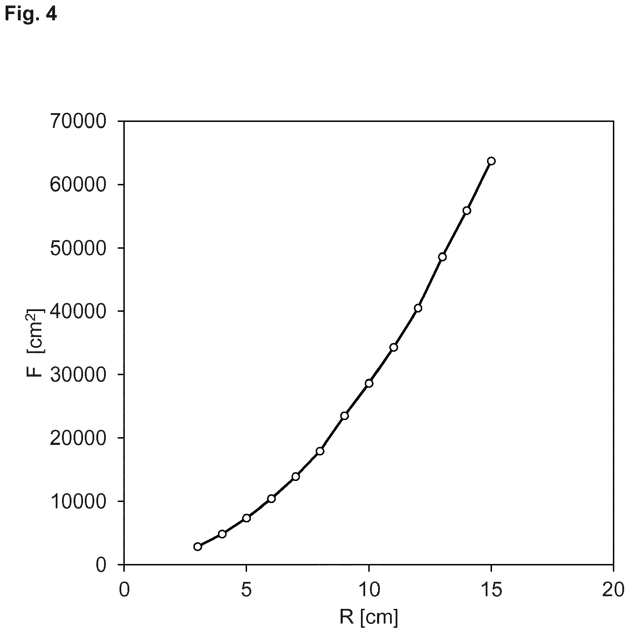

Fig. 4 zeigt wie stark die Wachstumsoberfläche F im Gefäß 10, im Verhältnis zum Radius R des Gefäßes 10 anwächst. In diesem Beispiel weist der Zylinderbereich 16 eine Höhe von 16 cm bis 20 cm auf und umfasst 20 Windungen, bei 0,8 cm Abstand zwischen den Wänden 50.
Fig. 5 zeigt die Verwendung der erfindungsgemäßen Zellkultur-Flasche 1 an Stelle eines Rührers in einem schräg stehenden klassischen Bioreaktor.

### Bezugszeichenliste

- 1: Zellkultur-Flasche
- 2: Reaktionskessel
- 3: Sonden
- 4: Zufluss
- 5: Abfluss
- 6: Fluid
- 7: Drehmechanismus des Rührkesselreaktor
- 10: Gefäß
- 11: Öffnung des Gefäßes 10
- 12: Bodenbereich
- 14: Deckelbereich
- 16: Zylinderbereich
- 30: archimedische Schraube
- 40: Mittelrohr
- 41: erste Öffnung des Mittelrohres 40
- 42: zweite Öffnung des Mittelrohres 40
- 43: dritte Öffnung des Mittelrohres 40
- 50: Wand
- α: Anstiegswinkel der archimedischen Schraube 30
- β: Anstiegswinkel des Gefäßes 10
- D: Durchmesser des Gefäßes 10

## Patentansprüche

1. Zellkultur-Flasche (1) umfassend
• ein Gefäß (10), umfassend drei Teilbereiche: einen Bodenbereich (12), einen Deckelbereich (14) und einen dazwischen angeordneten Zylinderbereich (16),
• eine im Zylinderbereich (16) des Gefäßes (10) innenliegend angeordnete archimedische Schraube (30) mit einem entlang ihrer Mittelachse angeordneten flüssigkeitsdurchströmbaren Mittelrohr (40),
• wenigstens eine konzentrisch um das Mittelrohr (40) angeordnete Wand (50), welche sich über den gesamten Zylinderbereich (16) des Gefäßes (10) erstreckt,
**dadurch gekennzeichnet, dass** die Zellkultur-Flasche einstückig ausgebildet ist und wobei das Mittelrohr (40) so ausgebildet ist, dass es eine erste Öffnung (41) an der Außenseite des Gefäßes (10) im Deckelbereich (14) aufweist und eine zweite Öffnung (42) im Inneren des Gefäßes (10) an der Unterseite des Zylinderbereiches (16) aufweist und wobei die archimedische Schraube (30) einstückig mit dem Zylinderbereich (16) des Gefäßes (10) und der wenigstens einen Wand (50) verbunden ist, wobei die archimedische Schraube (30) und die wenigstens eine Wand (50) so angeordnet sind, so dass bei einer Drehung der Zellkultur-Flasche (1) ein Fluid entlang der archimedischen Schraube (30) von der Unterseite des Zylinderbereiches (16) in den Deckelbereich (14) des Gefäßes (10) gelangen kann.

2. Zellkultur-Flasche (1) gemäß Anspruch 1 **dadurch gekennzeichnet, dass** das Gefäß (10) eine Öffnung (11) im Deckelbereich (14) außerhalb des Mittelrohres (40) aufweist.

3. Zellkultur-Flasche (1) gemäß Anspruch 1 oder 2 **dadurch gekennzeichnet, dass** die Zellkultur-Flasche (1) eine Beschichtung umfassend Polysaccharide und /oder Peptide aufweist, wobei diese auf der Oberfläche der archimedischen Schraube (30), der wenigstens einen Wand (50) und/oder der Innenseite des Gefäßes (10) angebracht ist, sodass Zellen an den inneren Oberflächen der Zellkultur-Flasche (1) anhaften und wachsen können.

4. Zellkultur-Flasche (1) gemäß einem der vorangegangenen Ansprüche **dadurch gekennzeichnet, dass** sie einen geschlossenen Bodenbereich (12) aufweist, sodass die Zellkultur-Flasche (1) an der Unterseite des Zylinderbereiches (16) geschlossen ausgebildet ist, wobei der Bodenbereich (12) einstückig mit dem Zylinderbereich (16) verbunden ist.

5. Zellkultur-Flasche (1) gemäß einem der vorangegangenen Ansprüche **dadurch gekennzeichnet, dass** die Zellkultur-Flasche (1) einen Drehmechanismus zum Drehen des Gefäßes (10) aufweist, wobei dieser Drehmechanismus am Bodenbereich (12) des Gefäßes (10) anbringbar ist.

6. Zellkultur-Flasche (1) gemäß einem der vorangegangenen Ansprüche **dadurch gekennzeichnet, dass** die Zellkultur-Flasche (1) im Mittelrohr (40) einen Filter aufweist.

7. Zellkultur-Flasche (1) gemäß einem der vorangegangenen Ansprüche **dadurch gekennzeichnet, dass** das Mittelrohr (40) eine dritte Öffnung (43) aufweist, wobei sich diese an der Kontaktstelle des Mittelrohres (40) mit dem Deckelbereich (14) des Gefäßes (10) befindet, sodass Fluid vom Bodenbereich (12) über die archimedische Schraube (30) zum Deckelbereich (14) des Gefäßes (10) gelangen und über die dritte Öffnung (43) in das Mittelrohr 40 wieder an die Unterseite des Zylinderbereiches (16) des Gefäßes (10) zurückfließen kann.

8. Zellkultur-Flasche (1) gemäß einem der vorangegangenen Ansprüche **dadurch gekennzeichnet, dass** sie aus einem Material gefertigt ist, das zur additiven Fertigung geeignet ist.

9. Zellkultur-Flasche (1) gemäß Anspruch 8 **dadurch gekennzeichnet, dass** das Material ausgewählt ist aus der Gruppe Acrylnitril-Butadien-Styrol (ABS), Acrylester-Styrol-Acrylnitril (ASA), High-Impact Polystyrene (HIPS), thermoplastische Elastomere (TPE), Polycarbonate (PC), Polylactide (PLA), Polyethylenterephthalat (PET), Polyethylenterephthalat Glycol (PETG), Polymethylmethacrylat (PMMA) oder Polyvinylalkohol (PVA oder PVOH.

10. Kultivierungssystem, **dadurch gekennzeichnet, dass** es eine Zellkultur-Flasche (1) gemäß einem der Ansprüche 1 bis 9 umfasst.

11. Verwendung einer Zellkultur-Flasche (1) gemäß einem der Ansprüche 1 bis 9 zur Kultivierung von Zellen.

12. Verfahren zur Kultivierung von Zellen in einer Zellkultur-Flasche (1) gemäß der Ansprüche 1-9 umfassend wenigstens die Schritte:
a) Befüllen der Zellkultur-Flasche (1) mit Fluid
b) Zugabe der zu kultivierenden Zellen in die Zellkultur-Flasche (1)
c) Drehen der Zellkultur-Flasche (1) so dass sich das Fluid verteilt und die Zellen bedeckt, so dass sich die Zellen im Gefäß (10), in der archimedischen Schraube (30), an der wenigstens einen Wand (50) und dem Mittelrohr (40) festsetzen und teilen können
d) Entfernen des Fluids bei einer bestimmten Zelldichte
e) Zugabe eines Puffers in die Zellkultur-Flasche (1), um die Zellen zu waschen
f) Ablösen der Zellen vom Gefäß (10), der archimedischen Schraube (30), der wenigstens einen Wand (50) und dem Mittelrohr (40)
g) Stoppen der Ablösereaktion durch Zugabe von frischem Fluid
h) Entnahme des Fluids mit losgelösten kultivierten Zellen

## Claims

1. Cell culture bottle (1) comprising
• a vessel (10) comprising three sections: a bottom section (12), a lid section (14) and a cylinder section (16) arranged therebetween,
• an Archimedean screw (30) arranged internally in the cylinder region (16) of the vessel (10) and having a central tube (40) through which liquid can flow arranged along its central axis,
• at least one wall (50) arranged concentrically around the central tube (40) and extending over the entire cylindrical region (16) of the vessel (10),
**characterised in**
**that** the cell culture flask is formed in one piece and wherein the central tube (40) is formed to have a first opening (41) on the outside of the vessel (10) in the lid region (14) and a second opening (42) in the interior of the vessel (10) on the underside of the cylinder region (16) and wherein the Archimedean screw (30) is integrally connected to the cylinder region (16) of the vessel (10) and the at least one wall (50), wherein the Archimedean screw (30) and the at least one wall (50) are arranged such that, upon rotation of the cell culture flask (1), a fluid can pass along the Archimedean screw (30) from the underside of the cylinder portion (16) into the lid portion (14) of the vessel (10).

2. Cell culture bottle (1) according to claim 1, **characterised in that** the vessel (10) has an opening (11) in the lid region (14) outside the central tube (40).

3. Cell culture bottle (1) according to claim 1 or 2 **characterized in that** the cell culture bottle (1) has a coating comprising polysaccharides and/or peptides applied to the surface of the Archimedes screw (30), the at least one wall (50) and/or the inside of the vessel (10) so that cells can adhere and grow on the inner surfaces of the cell culture bottle (1).

4. Cell culture bottle (1) according to any one of the preceding claims, **characterized in that** it comprises a closed bottom portion (12) such that the cell culture bottle (1) is closed at the bottom of the cylinder portion (16), the bottom portion (12) being integrally connected to the cylinder portion (16).

5. Cell culture bottle (1) according to any one of the preceding claims, **characterized in that** the cell culture bottle (1) comprises a rotating mechanism for rotating the vessel (10), said rotating mechanism being attachable to the bottom portion (12) of the vessel (10).

6. Cell culture bottle (1) according to any one of the preceding claims, **characterised in that** the cell culture bottle (1) has a filter in the central tube (40).

7. Cell culture bottle (1) according to any one of the preceding claims, **characterised in that** the central tube (40) has a third opening (43), this opening being located at the point of contact of the central tube (40) with the lid region (14) of the vessel (10), so that fluid can pass from the bottom portion (12) via the Archimedean screw (30) to the lid region (14) of the vessel (10) and flow back via the third opening (43) in the central tube (40) to the underside of the cylinder region (16) of the vessel (10).

8. Cell culture bottle (1) according to any one of the preceding claims, **characterized in that** it is made of a material suitable for additive manufacturing.

9. Cell culture bottle (1) according to claim 8, **characterised in that** the material is selected from the group acrylonitrile-butadiene-styrene (ABS), acrylic ester-styrene-acrylonitrile (ASA), high-impact polystyrene (HIPS), thermoplastic elastomers (TPE), polycarbonates (PC), polylactides (PLA), polyethylene terephthalate (PET), polyethylene terephthalate glycol (PETG), polymethyl methacrylate (PMMA) or polyvinyl alcohol (PVA or PVOH).

10. Cultivation system, **characterised in that** it comprises a cell culture bottle (1) according to any one of claims 1 to 9.

11. Use of a cell culture bottle (1) according to any one of claims 1 to 9 for the cultivation of cells.

12. A method for culturing cells in a cell culture bottle (1) according to claims 1-9 comprising at least the steps of:
a) filling the cell culture bottle (1) with fluid
b) adding the cells to be cultured into the cell culture bottle (1)
c) rotating the cell culture bottle (1) so that the fluid is distributed and covers the cells, allowing the cells to settle and divide in the vessel (10), in the Archimedes screw (30), on the at least one wall (50) and the central tube (40)
d) removing the fluid at a certain cell density
e) adding a buffer to the cell culture flask (1) to wash the cells
f) detaching the cells from the vessel (10), the Archimedes screw (30), the at least one wall (50) and the central tube (40)
g) stopping the detachment reaction by adding fresh fluid
h) removing the fluid with detached cultured cells.

## Revendications

1. flacon de culture cellulaire (1) comprenant
- un récipient (10) comprenant trois sections : une section de fond (12), une section de couvercle (14) et une section de cylindre (16) disposée entre elles
- une vis d'Archimède (30) disposée à l'intérieur de la zone cylindrique (16) du récipient (10) et ayant un tube central (40) à travers lequel le liquide peut s'écouler disposé le long de son axe central,
- au moins une paroi (50) disposée concentriquement autour du tube central (40) et s'étendant sur toute la région cylindrique (16) du récipient (10),
**caractérisé en ce que** le flacon de culture cellulaire est formé en une seule pièce et dans lequel le tube central (40) est formé pour avoir une première ouverture (41) sur l'extérieur du récipient (10) dans la région du couvercle (14) et une seconde ouverture (42) à l'intérieur du récipient (10) sur le côté inférieur de la région cylindrique (16) et dans lequel la vis d'Archimède (30) est reliée intégralement à la région cylindrique (16) du récipient (10) et à au moins une paroi (50), dans lequel la vis d'Archimède (30) et la au moins une paroi (50) sont disposées de telle sorte que, lors de la rotation du flacon de culture cellulaire (1), un fluide peut passer le long de la vis d'Archimède (30) depuis le côté inférieur de la partie cylindrique (16) dans la partie de couvercle (14) du récipient (10).

2. Flacon de culture cellulaire (1) selon la revendication 1, **caractérisé en ce que** le récipient (10) présente une ouverture (11) dans la région du couvercle (14) à l'extérieur du tube central (40).

3. Flacon de culture cellulaire (1) selon la revendication 1 ou 2 **caractérisé en ce que** le flacon de culture cellulaire (1) a un revêtement comprenant des polysaccharides et/ou des peptides appliqués à la surface de la vis d'Archimède (30), de la au moins une paroi (50) et/ou de l'intérieur du récipient (10) de sorte que les cellules peuvent adhérer et se développer sur les surfaces intérieures du flacon de culture cellulaire (1).

4. Flacon de culture cellulaire (1) selon l'une quelconque des revendications précédentes, **caractérisé en ce qu'**il comprend une partie de fond fermée (12) de telle sorte que le flacon de culture cellulaire (1) soit fermé au fond de la partie cylindrique (16), la partie de fond (12) étant reliée d'un seul tenant à la partie cylindrique (16).

5. Flacon de culture cellulaire (1) selon l'une quelconque des revendications précédentes, **caractérisé en ce que** le flacon de culture cellulaire (1) comprend un mécanisme de rotation pour faire tourner le récipient (10), ledit mécanisme de rotation pouvant être fixé à la partie inférieure (12) du récipient (10).

6. Flacon de culture cellulaire (1) selon l'une quelconque des revendications précédentes, **caractérisé en ce que** le flacon de culture cellulaire (1) comporte un filtre dans le tube central (40).

7. Flacon de culture cellulaire (1) selon l'une quelconque des revendications précédentes, **caractérisé en ce que** le tube central (40) présente une troisième ouverture (43), cette ouverture étant située au niveau du point de contact du tube central (40) avec la zone de couvercle (14) du récipient (10), de sorte que le fluide peut passer de la zone de fond (12) via la vis d'Archimède (30) à la zone de couvercle (14) du récipient (10) et refluer via la troisième ouverture (43) du tube central (40) vers la face inférieure de la zone cylindrique (16) du récipient (10).

8. Flacon de culture cellulaire (1) selon l'une quelconque des revendications précédentes, **caractérisé en ce qu'**il est réalisé dans un matériau adapté à la fabrication additive.

9. Flacon de culture cellulaire (1) selon la revendication 8, **caractérisé en ce que** le matériau est choisi dans le groupe acrylonitrile-butadiène-styrène (ABS), ester acrylique-styrène-acrylonitrile (ASA), polystyrène à haute résistance aux chocs (HIPS), élastomères thermoplastiques (TPE), polycarbonates (PC), polylactides (PLA), polyéthylène téréphtalate (PET), polyéthylène téréphtalate glycol (PETG), polyméthacrylate de méthyle (PMMA) ou alcool polyvinylique (PVA ou PVOH).

10. Système de culture, **caractérisé en ce qu'**il comprend un flacon de culture cellulaire (1) selon l'une quelconque des revendications 1 à 9.

11. Utilisation d'un flacon de culture cellulaire (1) selon l'une quelconque des revendications 1 à 9 pour la culture de cellules.

12. Procédé de culture de cellules dans un flacon de culture cellulaire (1) selon les revendications 1 à 9, comprenant au moins les étapes suivantes:
a) remplir le flacon de culture cellulaire (1) de fluide
b) ajouter les cellules à cultiver dans le flacon de culture cellulaire (1)
c) faire tourner le flacon de culture cellulaire (1) de manière à ce que le fluide soit distribué et recouvre les cellules, permettant aux cellules de se déposer et de se diviser dans le récipient (10), dans la vis d'Archimède (30), sur la au moins une paroi (50) et le tube central (40)
d) retirer le fluide à une certaine densité cellulaire
e) ajouter un tampon au flacon de culture cellulaire (1) pour laver les cellules
f) détacher les cellules du récipient (10), de la vis d'Archimède (30), de l'au moins une paroi (50) et du tube central (40).
g) arrêter la réaction de détachement en ajoutant du fluide frais
h) éliminer le liquide contenant les cellules de culture détachées.
